# EUROPÄISCHE PATENTANMELDUNG

(11) **EP 4 005 462 A1**
(43) Veröffentlichungstag der Anmeldung: **01.06.2022**
(21) Anmeldenummer: 21208006.3
(22) Anmeldetag: 12.11.2021
(51) Int. Cl.: A61B 1/00

(54) **OPERATIONS-ASSISTENZ-SYSTEM UND VERFAHREN ZUR ERZEUGUNG VON STEUERSIGNALEN ZUR SPRACHSTEUERUNG EINER MOTORISCH GESTEUERT BEWEGBAREN ROBOTERKINEMATIK EINES DERARTIGEN OPERATIONS-ASSISTENZ-SYSTEMS**

(30) Priorität: 25.11.2020 DE 102020131232
(71) Anmelder: KHS GmbH, 44143 Dortmund (DE)
(72) Erfinder: GEIGER, Robert, 94526 Metten (DE)
(74) Vertreter: Glück Kritzenberger Patentanwälte PartGmbB

(57) **Zusammenfassung**

Die Erfindung betrifft ein Operations-Assistenz-System (1) zur Führung einer Endoskopkamera (20), welche über eine erste Operationsöffnung (11) in einem Operationsraum (12) eines Patientenkörpers (10) zumindest abschnittsweise einführbar und gesteuert bewegbar ist, umfassend eine Endoskopkamera (20) zur Erfassung von Bildern (B) des Operationsraumes (12) in Form von Bilddaten (BD) und eine die Endoskopkamera (20) mittels einer Hilfsinstrumentenhalterung (6) freiendseitig aufnehmende Roboterkinematik (3, 4, 5), die zur Führung der Endoskopkamera (20) im Operationsraum (12) motorisch gesteuert bewegbar ist, und zwar über von einer Steuereinheit (CU) erzeugte Steuersignale (SS), dass in der Steuereinheit (CU) zumindest eine Sprachsteuerroutine (SSR) ausgeführt wird, über die Sprachbefehle und/oder Sprachbefehlskombinationen (SB, SBK, OSB, SSB) in Form von Sprachdaten (SD) erfasst, ausgewertet und abhängig davon die Steuersignale (SS) erzeugt werden, und dass in der Steuereinheit (CU) zumindest eine Bilderfassungsroutine (BER) zur kontinuierlichen Erfassung der von der Endoskopkamera (20) bereitgestellten Bilddaten (BD) betreffend den Operationsraum (12) ausgeführt wird. Besonders vorteilhaft ist in der Steuereinheit (CU) zumindest eine Bildauswerteroutine (BAR) vorgesehen, mittels der die erfassten Bilddaten (BD) basierend auf statistischen und/oder selbstlernenden Methoden der künstlichen Intelligenz kontinuierlich ausgewertet und klassifiziert werden. Anschließend wird mittels der kontinuierlichen Auswertung und Klassifikation der Bilddaten (BD) objekt- und/oder szenebezogene Informationen (OI, SI) betreffend die von der Endoskopkamera (20) aktuell im Bild (B) erfasste Operationsszene ermittelt, und die erfassten Sprachdaten (SD) abhängig von den erfassten Objekt- und/oder szenebezogene Informationen (OI, SI) ausgewertet.

## Beschreibung

Die Erfindung betrifft ein Operations-Assistenz-System gemäß dem Oberbegriff des Patentanspruches 1 und ein Verfahren zur Erzeugung von Steuersignalen zur Sprachsteuerung einer motorisch gesteuert bewegbaren Roboterkinematik eines Operations-Assistenz-Systems gemäß dem Oberbegriff des Patentanspruches 14.

Operations-Assistenz-Systeme, insbesondere zur Unterstützung von medizinischen Eingriffen oder Operationen, insbesondere minimal-invasiven Operationen sind hinlänglich bekannt. Diese dienen zur Verbesserung der Sicherheit, der Effizienz und der Ergebnisqualität in modernen chirurgischen Operationssälen. Insbesondere im Bereich der technologiegetriebenen minimal-invasiven Chirurgie sind derartige Assistenzsysteme von besonderer Bedeutung.

Operations-Assistenz-Systeme bzw. Assistenzsysteme werden häufig zur Führung von medizinischen Hilfsinstrumenten, wie z.B. Kamerasystemen, insbesondere so genannten Endoskopkameras verwendet. Aus der DE 10 2007 019363 A1 ist beispielsweise ein Operations-Assistenz-System bekannt, mittels dem beispielsweise ein eine Kameraeinheit aufweisendes Endoskop bzw. eine Endoskopkamera gesteuert geführt wird. Das Operations-Assistenz-System weist hierzu eine Roboterkinematik auf, die mittels mehrerer Antriebseinheiten gesteuert antreibbar ausgebildet ist. Mittels dieser Roboterkinematik kann eine an einer Instrumentenhalterung aufgenommene Endoskopkamera in einem dreidimensionalen Raum, insbesondere dessen freies Kameraende in einem inneren Operationsraum im Rahmen eines minimal-invasiven operativen Eingriffs gesteuert bewegt werden. Die Roboterkinematik umfasst hierzu beispielsweise wenigstens eine Tragsäule, wenigstens zwei Roboterarme und wenigstens einen die Instrumentenhalterung aufnehmenden Instrumententräger.

Aus der DE 10 2008 016 146 B4 ist ferner ein Verfahren zur Führung einer Endoskopkamera mittels eines derartigen Operations-Assistenz-Systems bekannt, und zwar abhängig von der manuellen Betätigung zumindest einer Funktionstaste eines Bedienelements. Auch sind bereits Assistenzsysteme bekannt geworden, welche zur automatisierten Nachführung einer Endoskopkamera abhängig von der aktuellen Position eines chirurgischen Instrumentes eingerichtet sind.

Steuersysteme für derartige Operations-Assistenz-Systeme können auch über eine Sprachsteuerung verfügen wie beispielsweise aus der DE 10 2017 101 782 A1 und der WO 2013/ 186 794 A2 bekannt geworden ist. Mittels einer derartigen Sprachsteuerung kann der Operateur über Sprachbefehle die Führung des Hilfsinstruments steuern. Insbesondere ist aus der DE 20 2013 012 276 U1 ein chirurgisches Steuersystem bekannt, bei dem neben einer Steuerung mittels Sprachbefehlen, auch die von einer Bilderfassungsvorrichtung erfassten Bilddaten des Operationsraumes erfasst und anschließend mittels einer Bildverarbeitung ausgewertet werden. Jedoch ist hier weiterhin eine Steuerung der Führung des Hilfsinstruments durch den Operateur oder eines Kameraassistenten gemäß den Weisungen des Operateurs erforderlich, der abhängig von dem ihm auf einer Monitoreinheit angezeigten Bildaufnahmen bzw. Bildern die Steuerung des Hilfsinstrumentes wie beispielsweise einer Endoskopkamera selbst vornimmt. Nachteilig basieren bekannte Sprachsteuerungen auf fest vorgegebenen Befehlskatalogen, welche vom Operateur oder Kameraassistenten beherrscht werden müssen, um eine zuverlässige Steuerung zu gewährleisten. Eine intuitive Bedienbarkeit durch den Operateur oder Kameraassistenten ist mit den bekannten Sprachsteuersystemen nicht möglich.

Auch sind aus dem Stand der Technik bereits Verfahren und Vorrichtungen zum Erkennen von Bildmustern oder Objekten in Bildern sowie auch zum Erkennen einer Abfolge phonetischer Laute oder Zeichen in Sprachsignalen bekannt. Beispielsweise wird in der DE 100 06 725 ein Verfahren zur Sprachverarbeitung unter Verwendung eines neuronalen Netzwerks beschrieben, welches eine Spracheingabe mittels unformatierter Sprachbefehle oder Sprachkommandos ermöglicht.

Ausgehend davon ist es Aufgabe der Erfindung, ein Operations-Assistenz-System, insbesondere für medizinische Interventionen oder Operationen sowie ein zugehöriges Verfahren zur Erzeugung von Steuersignalen zur Steuerung der Roboterkinematik eines derartigen Operations-Assistenz-Systems mittels Sprachbefehlen aufzuzeigen, welches den Operateur von der Kameraführungsaufgabe weitgehend entlastet, insbesondere sich durch eine intuitive Bedienbarkeit auszeichnet und die Kameraführung bei minimal-invasiven Eingriffen für den Operateur vereinfacht.

Die Aufgabe wird durch ein Operations-Assistenz-System mit den Merkmalen des Oberbegriffes des Patentanspruches 1 durch dessen kennzeichnende Merkmale gelöst. Ferner wird die Aufgabe ausgehend von einem Verfahren mit den Merkmalen des Oberbegriffes des Patentanspruches 14 durch dessen kennzeichnende Merkmal gelöst.

Ein wesentlicher Aspekt des erfindungsgemäßen Operations-Assistenz-Systems ist darin zu sehen, dass in der Steuereinheit zumindest eine Bildauswerteroutine ausgeführt wird bzw. vorgesehen ist, mittels der die erfassten Bilddaten basierend auf statistischen und/oder selbstlernenden Methoden der künstlichen Intelligenz kontinuierlich ausgewertet und klassifiziert werden, dass mittels der kontinuierlichen Auswertung und Klassifikation der Bilddaten objekt- und/oder szenebezogene Informationen betreffend die von der Endoskopkamera aktuell im Bild erfasste Operationsszene ermittelt werden, und dass die erfassten Sprachdaten abhängig von den erfassten objekt- und/oder szenebezogene Informationen ausgewertet werden. Besonders vorteilhaft ist durch die erfindungsgemäß kombinierte Erfassung und Auswertung von auf eine Operationsszene bezogenen Bild- und Sprachdaten eine intuitivere Sprachsteuerung der Endoskopkamera durch den Operateur möglich, da dieser vom Kamerabild intuitiv ableitbare Sprachbefehle und/oder Sprachbefehlskombinationen zur Steuerung der Endoskopkamera einsetzen kann. Der Operateur kann beispielsweise zur Sprachsteuerung Sprachkommandos bzw. Sprachbefehle oder Sprachbefehlskombinationen verwenden, welche auf einzelne im Bild dargestellte Objekte und/oder deren Position und/oder deren Orientierung im Bild Bezug nehmen.

Weiterhin vorteilhaft umfasst die Bildauswerteroutine zur Auswertung der erfassten Bilddaten ein neuronales Netz mit Muster- und/oder Farberkennungsalgorithmen. Die Muster- und/oder Farberkennungsalgorithmen sind vorzugsweise zur Erfassung oder Detektion von im Bild vorhandener Objekte oder Teile davon, insbesondere von Operationsinstrumenten oder anderen medizinischen Hilfsmitteln oder Organen eingerichtet und trainiert. Vorteilhaft sind die genannten Algorithmen Bestandteil eines neuronalen Netzwerks, welches über die Verarbeitung von einer Vielzahl von Trainingsdatensätzen trainiert ist.

In einer bevorzugten Ausführungsvariante ist die Sprachsteuerroutine zur Auswertung der Sprachdaten basierend auf statistischen und/oder selbstlernenden Methoden der künstlichen Intelligenz eingerichtet. Die Sprachsteuerroutine umfasst beispielsweise zur Auswertung der Sprachdaten ein neuronales Netz mit Laut- und/oder Silbenerkennungsalgorithmen, wobei die Laut- und/oder Silbenerkennungsalgorithmen zur Erfassung von in den Sprachdaten enthaltenen Laute, Silben, Wörtern, Wortpausen und/oder Kombinationen davon eingerichtet sind. Durch wird können auch nicht vorgegebene oder unformatierte Sprachbefehle und/oder Sprachbefehlskombinationen, und zwar bild- und/oder szenebezogenen Sprachbefehle und/oder Sprachbefehlskombinationen zur Steuerung der Endoskopkamera verwendet werden.

Weiterhin vorteilhaft ist die in der Steuereinheit ausgeführte Sprachsteuerroutine zur Erfassung und Auswertung von objekt- und/oder szenebezogener Sprachbefehlen abhängig von den objekt- und/oder szenebezogenen Informationen eingerichtet. Durch die Berücksichtigung der objekt- und/oder szenebezogenen Informationen können neuartige, auf die im Bild B dargestellte Operationsszene inhaltlich Bezug nehmende Sprachbefehle zur Erzeugung von Steuersignalen automatisiert verarbeitet werden. Dadurch erhöht sich der zur Ansteuerung verwendbarer Steuerbefehle für den Operateur wesentlich, was zu einer intuitiveren Sprachsteuerung führt.

In einer vorteilhaften Weiterbildung der Erfindung wird abhängig von den erfassten objekt-und/oder szenebezogene Sprachbefehlen zumindest ein zugeordnetes Steuersignal erzeugt, über welche zumindest die Bewegung des Endoskopkamera richtungs-, geschwindigkeits-und/oder betragsmäßig gesteuert wird. Auch kann die Sprachsteuerroutine zur Erfassung und Auswertung objekt- und/oder szenebezogenen Informationen auch Richtungs- und/oder Geschwindigkeitsinformationen und/oder zugehörige Betragsinformation in den Sprachdaten ausgebildet sein. Dadurch wird die Bedienerfreundlichkeit für den Operateur noch weiter erhöht.

Ebenfalls vorteilhaft kann die Endoskopkamera zur Erfassung eines zweidimensionalen oder eines dreidimensionalen Bildes ausgebildet sein. Somit können neben herkömmlichen zweidimensionalen Endoskopkameras auch dreidimensionale Endoskopkameras zur Erfassung der Bilddaten Verwendung finden. Vorteilhaft kann mittels derartiger dreidimensionale Endoskopkameras auch eine Tiefeninformation gewonnen werden, die als weiterer Regel- oder Steuerparameter durch die Steuereinheit ausgewertet werden kann. Die dadurch gewonnene Tiefeninformation kann besonders vorteilhaft zur Steuerung bzw. Nachführung der Endoskopkamera verwendet werden. Beispielsweise kann ein vorgegebener Abstand zwischen dem freien Ende der Endoskopkamera und einer erfassten Instrumentenspitze ein Regel- oder Steuerparameter bilden.

Besonders vorteilhaft wird dem Bild mittels der Bildauswerteroutine ein zweidimensionales Bildkoordinatensystem oder ein dreidimensionales Bildkoordinatensystem zugeordnet. Zur Ermittlung der Lage oder Position eines Objektes im Bild werden die Koordinaten des Objektes oder zumindest eines Markers oder eines Markerpunktes des Objektes im Bildschirmkoordinatensystem bestimmt. Dadurch kann die Position des erkannten Objektes im Bildschirmkoordinatensystem exakt bestimmt werden und daraus Steuersignale zur Führung der Endoskopkamera im Raumkoordinatensystem berechnet werden.

In einer vorteilhaften Ausführungsvariante werden mittels der Bildauswerteroutine als Objekte oder Teile von Objekten im Bild dargestellte Operationsinstrumente und/oder Organe und/oder andere medizinische Hilfsmittel detektiert. Zur Detektion von Objekten oder Teilen von Objekten können besonders vorteilhaft ein oder mehrere Marker oder Markerpunkte eines Objektes mittels der Bildauswerteroutine detektiert werden, wobei als Marker oder Markerpunkte beispielsweise eine Instrumentenspitze, besondere Farb- oder Materialeigenschaften des Objektes und/oder ein Gelenk zwischen einem Manipulator und dem Instrumentenschaft eines Operationsinstruments Verwendung finden können. Bevorzugt werden mittels der Bildauswerteroutine die detektierten Marker oder Markerpunkte zur Klassifizierung der Operationsszene und/oder der darin aufgefundenen Objekte(n) ausgewertet und abhängig davon die objektbezogenen und/oder szenebezogenen Informationen bestimmt werden. Anschließend werden die von der Bildauswerteroutine bestimmten objektbezogenen und/oder szenebezogenen Informationen an die Sprachsteuerroutine übergeben.

Ebenfalls ist Gegenstand der Erfindung ein Verfahren zur Erzeugung von Steuersignalen zur Ansteuerung einer motorisch gesteuert bewegbaren Roboterkinematik eines Operations-Assistenz-Systems zur Führung einer Endoskopkamera, bei dem die Endoskopkamera mittels einer Hilfsinstrumentenhalterung freiendseitig an der Roboterkinematik angeordnet ist, wobei die Endoskopkamera über eine erste Operationsöffnung zumindest abschnittsweise in den Operationsraum eines Patientenkörpers einführbar ist und in einer Steuereinheit zumindest eine Sprachsteuerroutine zur Erzeugung der Steuersignale ausgeführt wird. Vorteilhaft werden mittels der Sprachsteuerroutine Sprachbefehle und/oder Sprachbefehlskombinationen in Form von Sprachdaten erfasst, ausgewertet und abhängig davon die Steuersignale erzeugt und in der Steuereinheit zumindest eine Bilderfassungsroutine zur kontinuierlichen Erfassung der von der Endoskopkamera bereitgestellten Bilddaten betreffend den Operationsraum ausgeführt wird. Erfindungsgemäß werden mittels einer in der Steuereinheit ausgeführten Bildauswerteroutine die erfassten Bilddaten basierend auf statistischen und/oder selbstlernenden Methoden der künstlichen Intelligenz kontinuierlich ausgewertet und klassifiziert und mittels der kontinuierlichen Auswertung und Klassifikation der Bilddaten objekt- und/oder szenebezogene Informationen betreffend die von der Endoskopkamera aktuell im Bild erfasste Operationsszene ermittelt, wobei die erfassten Sprachdaten abhängig von den erfassten objekt- und/oder szenebezogene Informationen ausgewertet werden. Durch das erfindungsgemäße Verfahren ergibt sich vorteilhaft für den Operateur eine intuitivere, vom aktuellen Kamerabild abgeleitete Sprachsteuerung der Endoskopkamera.

Besonders vorteilhaft werden in der Bildauswerteroutine die erfassten Bilddaten mittels Muster- und/oder Farberkennungsalgorithmen eines neuronalen Netzes ausgewertet, wobei mittels der Muster- und/oder Farberkennungsalgorithmen die im Bild dargestellten Objekte oder Teile von Objekten, insbesondere Operationsinstrumenten oder anderen medizinischen Hilfsmitteln oder Organen detektiert werden. Die Muster- und/oder Farberkennungsalgorithmen sind Teil eines "trainierten" neuronalen Netzes, welches eine zuverlässige Auswertung der Bilddaten in Echtzeit ermöglicht.

In einer vorteilhaften Weiterbildung des erfindungsgemäßen Verfahrens werden zur Detektion der Objekte oder Teile von Objekten ein oder mehrere Marker oder Markerpunkte eines Objektes mittels der Bildauswerteroutine detektiert, wobei als Marker oder Markerpunkte beispielsweise eine Instrumentenspitze, besondere Farb- oder Materialeigenschaften des Objektes und/oder ein Gelenk zwischen einem Manipulator und dem Instrumentenschaft eines Operationsinstruments verwendet werden. Vorteilhaft werden mittels der Bildauswerteroutine die detektierten Marker oder Markerpunkte zur Klassifizierung der Operationsszene und/oder der darin aufgefundenen Objekte ausgewertet und abhängig davon die objektbezogenen und/oder szenebezogenen Informationen bestimmt.

Weiterhin vorteilhaft werden in der Sprachsteuerroutine die erfassten Sprachdaten mittels Laut- und/oder Silbenerkennungsalgorithmen eines neuronalen Netzes ausgewertet. Vorzugsweise werden mittels der Laut- und/oder Silbenerkennungsalgorithmen in den Sprachdaten enthaltene Laute, Silben, Wörtern, Wortpausen und/oder Kombinationen davon erfasst. Die von der Bildauswerteroutine bestimmten objektbezogenen und/oder szenebezogenen Informationen werden an die Sprachsteuerroutine übergeben. In der Sprachsteuerroutine werden objekt- und/oder szenebezogene Sprachbefehle erfasst und abhängig von den übergebenen objekt- und/oder szenebezogene Informationen ausgewertet.

In einer weiteren vorteilhaften Ausführungsvariante wird dem Bild mittels der Bildauswerteroutine ein zweidimensionales Bildkoordinatensystem zugeordnet und zur Lage-oder Positionsbestimmung eines Objektes im Bild die Koordinaten des Objektes oder zumindest eines Markers oder eines Markerpunktes des Objektes im Bildschirmkoordinatensystem bestimmt. Hierdurch ist eine zuverlässige Positionsbestimmung und davon abgeleitete Erzeugung von zugehörigen Steuersignalen möglich.

Die Ausdrucke "näherungsweise", "im Wesentlichen" oder "etwa" bedeuten im Sinne der Erfindung Abweichungen vom jeweils exakten Wert um +/- 10%, bevorzugt um +/- 5% und/oder Abweichungen in Form von für die Funktion unbedeutenden Änderungen.

Weiterbildungen, Vorteile und Anwendungsmöglichkeiten der Erfindung ergeben sich auch aus der nachfolgenden Beschreibung von Ausführungsbeispielen und aus den Figuren. Dabei sind alle beschriebenen und/oder bildlich dargestellten Merkmale für sich oder in beliebiger Kombination grundsätzlich Gegenstand der Erfindung, unabhängig von ihrer Zusammenfassung in den Ansprüchen oder deren Rückbeziehung. Auch wird der Inhalt der Ansprüche zu einem Bestandteil der Beschreibung gemacht.

Die Erfindung wird im Folgenden anhand der Figuren an Ausführungsbeispielen näher erläutert. Es zeigen:
- Fig. 1: beispielhaft eine perspektivische Seitenansicht eines Operations-Assistenz-Systems,
- Fig. 2: beispielhaft einen schematischen Schnitt durch einen Patientenkörper mit einer im Operationsraum zumindest abschnittsweise aufgenommenen Endoskopkamera sowie einem medizinischen Operationsinstrument,
- Fig. 3: beispielhaft ein von der Endoskopkamera erzeugtes Bild des Operationsraumes,
- Fig. 4: beispielhaft ein schematisches Blockschaltbild der Steuereinheit sowie der daran angeschlossenen Einheiten und der darin ausgeführten Steuer- und Auswerteroutinen,
- Fig. 5: beispielhaft ein Ablaufdiagramm eine Ausführungsvariante der erfindungsgemäßen Bildauswerteroutine und
- Fig. 6: beispielhaft ein Ablaufdiagramm eine Ausführungsvariante der erfindungsgemäßen Sprachsteuerroutine.

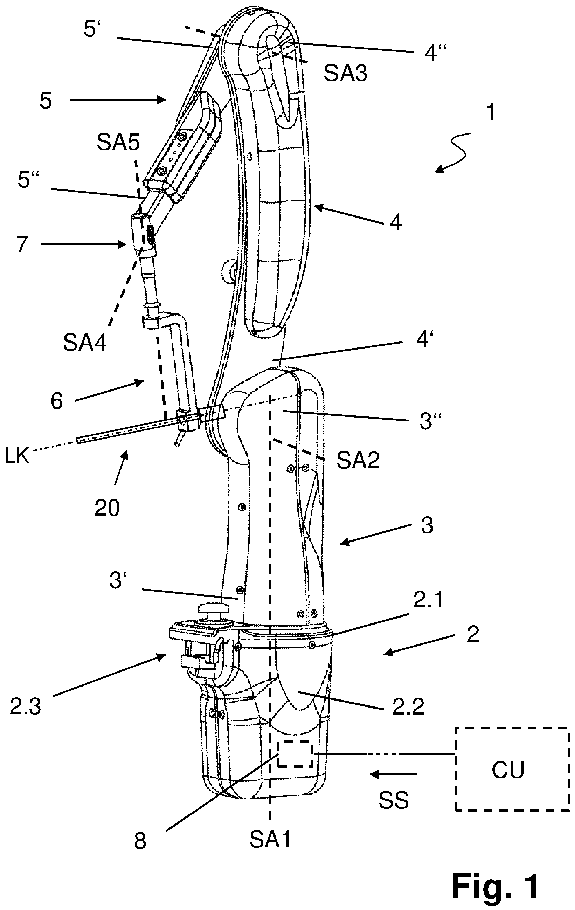

In Figur 1 ist beispielhaft ein Operations-Assistenz-System 1 zur Führung einer Endoskopkamera 20 bei medizinischen Eingriffen oder Operationen in einem Patientenkörper 10 dargestellt. Die Endoskopkamera 20 besteht im Wesentlichen aus einem Endoskop 21 und einer an dessen freien Ende angeordnete Kameraeinheit 22, welche sich vorzugsweise außerhalb des Patientenkörpers 10 befindet.

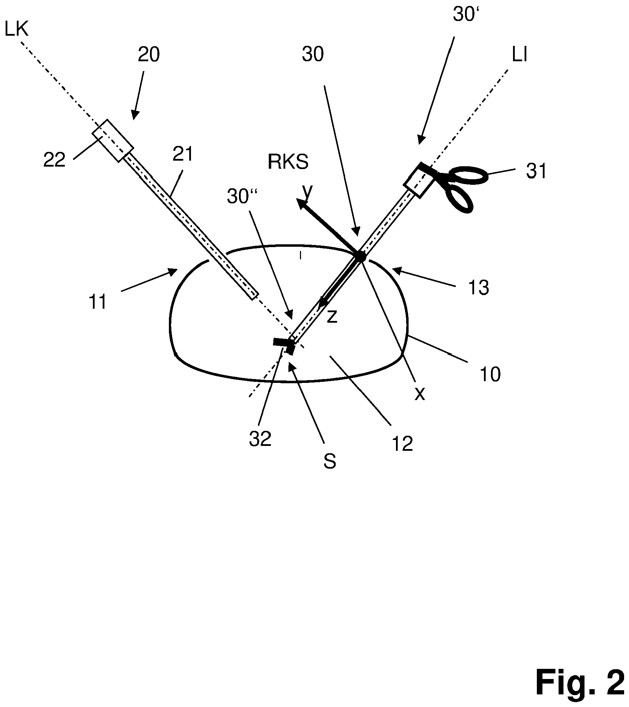

Derartige Endoskopkameras 20 werden bevorzugt bei minimal-invasiven Operationen eingesetzt. Hierzu werden diese über eine kleinformatige erste Operationsöffnung 11 in einen Operationsraum 12 innerhalb eines Patientenkörpers 10 eingeführt. Das eigentliche medizinische Operationsinstrument 30 zur Durchführung des medizinischen Eingriffs wird über eine zweite Operationsöffnung 13 in den Operationsraum 12 des Patientenkörpers 10 eingeführt. Die erste und zweite Operationsöffnung 11, 13 werden in der Literatur häufig auch als "Trokar" bzw. "Trokarpunkte" bezeichnet. In Figur 2 ist eine typische Operationssituation beispielhaft anhand eines Schnitts durch einen Patientenkörper 10 im minimal-invasiven Operationsbereich schematisch angedeutet.

Mittels der Endoskopkamera 20 bzw. dessen Kameraeinheit 22 werden dann kontinuierlich Bildaufnahmen bzw. Bilder B des Operationsraumes 12 in Form von Bilddaten BD, u.a. der im Operationsraum 12 befindlichen Spitze S des medizinischen Instrumentes 30 erzeugt, welche dem Operateur während des medizinischen Eingriffs bzw. der minimal-invasiven Operation auf einer nicht in den Figuren dargestellten Monitoreinheit angezeigt werden.

Der Operateur kann mittels den auf der Monitoreinheit dargestellten aktuellen Bilder B ("Livebilder") aus dem Operationsraum 12 den Verlauf der Operation überwachen und das im Bild B zu sehende Operationsinstrument 30 entsprechend führen. Um stets ein aktuelles und optimales Bild B des Operationsraumes 12, insbesondere des Operationsinstrumentes 30 vorliegen zu haben, ist für den Operateur eine möglichst intuitive Ansteuersteuerbarkeit und komfortable Nachführung der Endoskopkamera 22 von wesentlicher Bedeutung, insbesondere auch um während des operativen Eingriffs die Belastung des Operateurs durch die Kameraführungsaufgabe möglichst gering zu halten. Von besonderer Bedeutung ist hierbei die optimale Anzeige so genannten "Field of Interest" bezeichnet. Hierbei ist es besonders wünschenswert, dass sich die Spitze S des Operationsinstruments 30 im dem Operateur angezeigten Bild B im Zentrum bzw. der Bildmitte befindet.

Das erfindungsgemäße Operations-Assistenz-System 1 ermöglicht dem Operateur eine intuitive und bedienerfreundliche Bedienung bzw. Führung der Endoskopkamera 20 mittels einer kontextabhängigen "Sprachsteuerung". Ein entsprechend steuerbares Operations-Assistenz-System 1 besteht beispielsweise aus einer Basiseinheit 2 und einer ein System aus mehreren Armen, insbesondere Roboterarmen umfassenden Roboterkinematik, wobei die Roboterkinematik im vorliegenden Ausführungsbeispiel eine Tragsäule 3, einen ersten und zweiten Roboterarm 4, 5 und einen Hilfsinstrumententräger 6 aufweist. Der Hilfsinstrumententräger 6 ist beispielsweise am zweiten Roboterarm 5 angelenkt, und zwar mittels eines abgewinkelten Gelenkstückes 7. Der Hilfsinstrumententräger 6 ist beispielsweise zur direkten Aufnahme der Endoskopkamera 20 ausgebildet. Der beschriebene Aufbau der Roboterkinematik des Operations-Assistenz-Systems 1 ist aus der perspektivischen Darstellung des Operations-Assistenz-Systems 1 gemäß Figur 1 beispielhaft ersichtlich.

Die Basiseinheit 2 umfasst ferner beispielsweise eine Trägerplatte 2.1, ein vorzugsweise mehrteiliges Basisgehäuse 2.2 und zumindest ein Befestigungselement 2.3, mittels dem das vorzugsweise portabel ausgebildete Operations-Assistenz-System 1 bzw. die Basiseinheit 2 beispielsweise seitlich an einem Operationstisch (nicht in den Figuren dargestellt) befestigbar ist. Im Basisgehäuse 2.2 sind zumindest eine Steuereinrichtung 8 und ggf. weitere Funktionseinheiten aufgenommen, die ggf. mit einem nicht dargestellten Computersystem zusammenwirken.

Die Tragsäule 3 weist einen unteren und oberen Endabschnitt 3', 3" auf. Die Basiseinheit 2 des Operations-Assistenz-Systems 1 ist mit dem unteren Endabschnitt 3' der Tragsäule 3 der Roboterkinematik gesteuert schwenkbar um eine erste Schwenkachse SA1 verbunden. Die erste Schwenkachse SA1 verläuft hierbei vertikal zur Aufstellungsebene des Operations-Assistenz-Systems 1 bzw. zur Operationsebene bzw. Ebene eines Operationstisches.

Ferner weist der erste Roboterarm 4 einen ersten und zweiten Endabschnitt 4', 4" auf, wobei der erste Endabschnitt 4' des ersten Roboterarmes 4 mit dem der Basiseinheit 2 gegenüberliegenden oberen Endabschnitt 3" der Tragsäule 3 gesteuert schwenkbar um eine zweite Schwenkachse SA2 und der zweite Endabschnitt 4" des ersten Roboterarmes 4 mit einem ersten Endabschnitt 5' des zweiten Roboterarmes 5 gesteuert schwenkbar um eine dritte Schwenkachse SA3 verbunden ist.

Der zweite Roboterarm 5 weist einen zweiten, dem ersten Endabschnitt 5' gegenüberliegenden Endabschnitt 5" auf, an dem im vorliegenden Ausführungsbeispiel das abgewinkelte Gelenkstücks 7 drehbar um eine vierte Schwenkachse SA4 vorgesehen ist. Das abgewinkelte Gelenkstücks 7 ist zur drehbaren und lösbaren Aufnahme eines Anschlussabschnittes des Hilfsinstrumententrägers 6 ausgebildet, und zwar um eine fünfte Schwenkachse SA5. Das gegenüberliegende freie Ende des Hilfsinstrumententrägers 6 bildet eine Instrumentenaufnahme aus, welche vorzugsweise zur Aufnahme einer Endoskopkamera 20 ausgebildet ist.

Die erste Schwenkachse SA1 verläuft senkrecht zur Aufstellungsebene bzw. Operationsebene und die zweite und dritte Schwenkachse SA2, SA3 verlaufen parallel zueinander, wohingegen die erste Schwenkachse SA1 senkrecht zur zweiten oder dritten Schwenkachse SA2, SA3 orientiert ist.

Zum Antrieb der Roboterkinematik des Operations-Assistenz-Systems 1 sind mehrere, nicht in den Figuren dargestellte Antriebseinheiten vorgesehen, die über zumindest eine Steuereinrichtung 8 vorzugsweise unabhängig voneinander ansteuerbar ausgebildet sind. Die Antriebseinheiten sind vorzugsweise in der Basiseinheit 2, der Tragsäule 3 und/oder in den Roboterarmen 4 bis 6 integriert bzw. darin aufgenommen. Beispielsweise können die Antriebseinheiten durch hydraulische Antriebe oder elektrische Antriebe, insbesondere Linearmotoreinheiten oder Spindelmotoreinheiten gebildet sein.

Die zumindest eine Steuereinrichtung 8 ist vorzugsweise in der Basiseinheit 2 des Operations-Assistenz-Systems 1 aufgenommen und dient zur Erzeugung von Steuersignalen zur Ansteuerung der Antriebe bzw. Antriebseinheiten zum gesteuerten motorisches Schwenken der Roboterkinematik um die vorgegebenen Schwenkachsen SA1 bis SA5 und/oder Halten der Roboterkinematik in einer vorgegebenen Halteposition in einem kartesischen Koordinatensystem.

Die Tragsäule 3 erstreckt sich vertikal, und zwar im Wesentlichen entlang der ersten Schwenkachse SA1, d.h. ist näherungsweise um Ihre eigene Längsachse drehbar ausgebildet. Der erste und zweite Roboterarm 4, 5 erstrecken sich im Wesentlichen ebenfalls entlang einer Geraden, die vorzugsweise senkrecht zur zweiten bzw. dritten Schwenkachse SA2, SA3 verläuft. Im vorliegenden Ausführungsbeispiel weist zumindest der erste Roboterarm 4 eine leichte Krümmung auf.

Zur Einstellung der Ausgangsposition des Operations-Assistenz-Systems 1 bzw. zur Kalibrierung der Steuereinrichtung 8 in Bezug auf die erste Operationsöffnung 11 bzw. den Trokarpunkt, durch die Endoskopkamera 20 in den Operationsraum eingeführt wird, ist eine Registrierungsroutine vorgesehen, mittels der vor der Operation das Operations-Assistenz-System 1 beispielhaft dadurch registriert wird, dass ein in den Figuren nicht gezeigter Registriertaster an denjenigen Bereich des auf dem OP-Tisch bereits platzierten Patienten geführt wird, an dem die erste Operationsöffnung 11 zum Einführen der Endoskopkamera 20 vorgesehen ist. Nach dieser Kalibrierung ist das Operations-Assistenz-System 1 zur Führung der Endoskopkamera 20 einsatzbereit.

In Figur 2 ist beispielhaft eine schematische Seitenansicht einer in den Operationsraum 12 des Patientenkörpers 10 über die erste Operationsöffnung 11 eingeführten Endoskopkamera 20 dargestellt. Ferner zeigt Figur 2 ein über die zweite Operationsöffnung 13 in den Operationsraum 12 eingeführtes Operationsinstrument 30. Die zweite Operationsöffnung 13 bildet beispielsweise den Ursprung eines kartesischen Raumkoordinatensystems RKS mit den Raumachsen x, y, z.

In Figur 2 erstreckt sich beispielhaft die x-Achse des kartesischen Raumkoordinatensystems RKS senkrecht zur Zeichenebene, die y-Achse des kartesischen Raumkoordinatensystems RKS senkrecht zur Längsachse LI des Operationsinstruments 30, wohingegen die z-Achse entlang der Längsachse LI des Operationsinstruments 30 verläuft bzw. mit dieser zusammenfällt. Der Ursprung kommt im Bereich der zweiten Operationsöffnung 13 zu liegen. Vorteilhaft ist bei einer derartigen Orientierung des kartesischen Raumkoordinatensystems RKS eine Drehung um die Längsachse LI des medizinischen Instruments 30 jeweils eine Drehung um die z-Achse, welche beispielsweise eine vereinfachte Auswertung einer Drehbewegung um die Längsachse LI des Operationsinstruments 30 ermöglicht.

Das Operationsinstrument 30 weist beispielsweise an einem freien Ende 30' zumindest ein, vorzugsweise zwei Griffelemente 31 auf, die beispielsweise in Form zweier Griffringe mit jeweils anschließenden Verbindungsschaft ausgebildet sind. Über zumindest eines der Griffelemente 31 ist ein am gegenüberliegenden freien Ende 30" des medizinischen Instruments bzw. Operationsinstrumentes 30 angeordnetes Funktionselement 32, beispielsweise ein Greif- oder Schneidelement betätigbar. Das Funktionselement 32 bildet hierbei die Spitze S des Operationsinstruments 30 aus, welches sich während der Operation im Operationsraum 12 befindet und von der Endoskopkamera 20 erfasst wird. Das freie Ende 30' befindet sich außerhalb des Patientenkörpers 10 und bildet den Griffbereich des Operationsinstruments 30 aus.

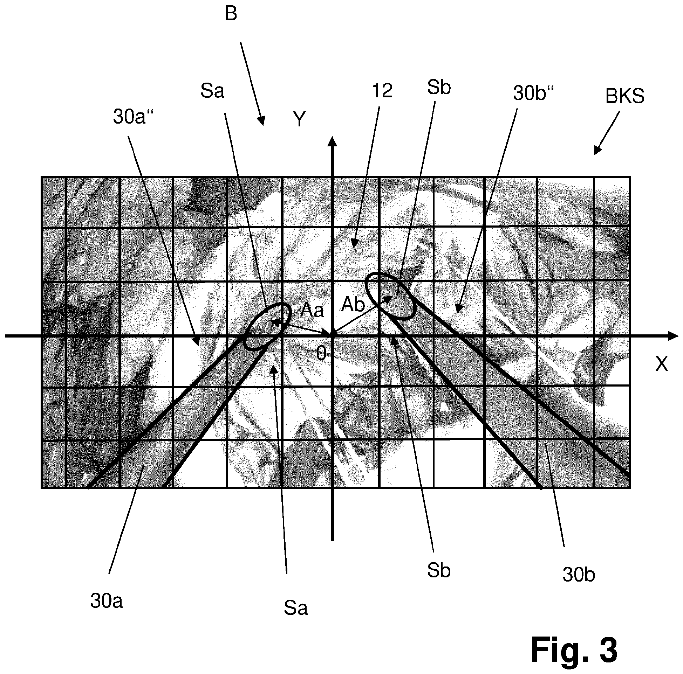

In Figur 3 ist beispielhaft ein Bild B des Operationsraumes 12 gezeigt, welches in Form von Bilddaten BD durch die Endoskopkamera 20 erfasst und beispielsweise in der dargestellten Form auf einer Monitoreinheit dem Operateur angezeigt wird. Das Bild B zeigt beispielhaft ein erstes und zweites Operationsinstrument 30a, 30b, und zwar die im Zentrum des Bildes B befindlichen Spitzen Sa, Sb der freien Enden 30a", 30b" der medizinischen Operationsinstrumente 30a, 30b, welche sich zumindest teilweise im Eingriff mit den dargestellten Organen befinden. Auch sind im Bild B zumindest Teile oder Abschnitte einzelner Organe erkennbar.

Zur Steuerung der Bewegung des Endoskopkamera 20, insbesondere Nachführung dessen nach einem Operationsinstrument 30, 30a, 30b im Operationsraum 12 weist das Operations-Assistenz-System 1 eine Steuereinheit CU auf, mittels der vorzugsweise auf das Raumkoordinatensystems RKS bezogene Steuersignale SS erzeugt werden. Diese werden dann an die Steuereinrichtung 8 übertragen, mittels der eine entsprechende Ansteuerung der Antriebe bzw. Antriebseinheiten der Roboterkinematik zum gesteuerten motorisches Schwenken der Tragsäule 3 und/oder der Roboterarme 4, 5 der Roboterkinematik erfolgt, um eine Dreh- und/oder Schwenkbewegung um die vorgegebenen Schwenkachsen SA1 bis SA5 und/oder ein Halten der Roboterkinematik in einer vorgegebenen Halteposition bezogen auf das Raumkoordinatensystems RKS zu bewirken. Die Steuereinrichtung 8 weist eine Robotersteuerroutine auf, die die zur Ansteuerung der unterschiedlichen Antriebseinheiten der Roboterkinematik erforderlichen Ansteuersignale erzeugt. Insbesondere werden mittels der Robotersteuerroutine abhängig von der übermittelten Steuersignalen SS ausgehend von einer bestehenden Ist-Position die von der Roboterkinematik anzufahrende Soll-Position jeweils bezogen auf das Raumkoordinatensystem RKS ermittelt und die hierzu erforderlichen Ansteuersignale erzeugt.

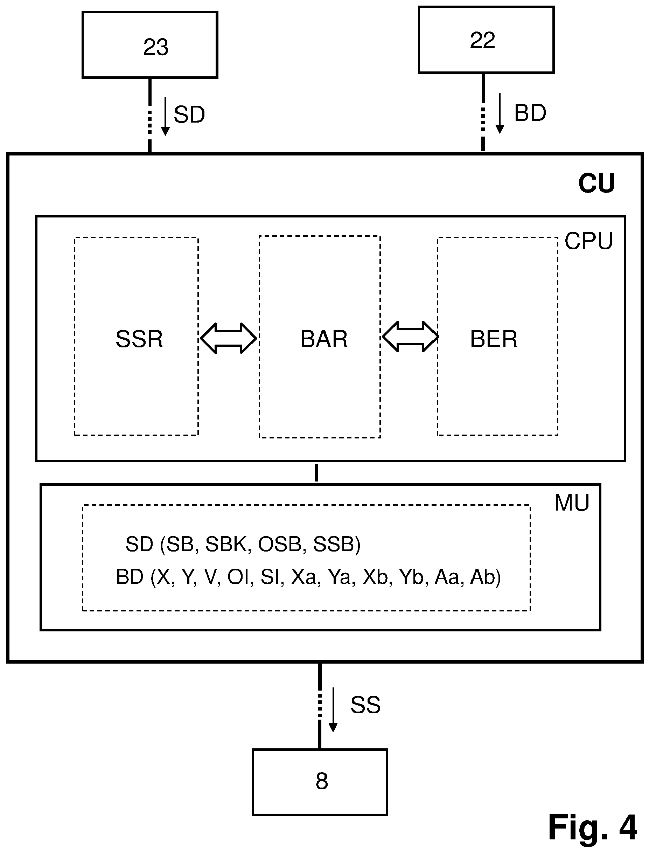

Die Steuereinheit CU umfasst beispielsweise zumindest eine Prozessoreinheit CPU und zumindest eine Speichereinheit MU. Vorzugsweise ist die Prozessoreinheit CPU durch zumindest einen oder mehrere leistungsfähige Mikroprozessoreinheiten gebildet. In der Prozessoreinheit CPU der Steuereinheit CPU wird zumindest eine Sprachsteuerroutine SSR ausgeführt, welche zur Erfassung und Auswertung der empfangenen Sprachbefehlen SB und/oder Sprachbefehlskombinationen SBK in Form von Sprachdaten SD sowie zu einer davon abhängigen Erzeugung von Steuersignalen SS ausgebildet ist. Die von der Sprachsteuerroutine SSR erzeugten Steuersignale SS werden anschließend von der Steuereinheit CU an die zur Ansteuerung der Roboterkinematik 3, 4, 5 vorgesehenen Steuereinrichtung 8 übertragen. Figur 4 zeigt beispielshaft ein schematisches Blockschaltbild einer erfindungsgemäßen Steuereinheit CU, welche im vorliegenden Ausführungsbespiel mit der Steuereinrichtung 8, der Kameraeinheit 22 der Endoskopkamera 20 und einer Mikrofoneinheit 23 verbunden ist.

Die Mikrofoneinheit 23 ist zur Erfassung von Sprachbefehlen SB und/oder Sprachbefehlskombinationen SBK vorgesehen. Diese kann beispielsweise in der Endoskopkamera 20 integriert oder in sonstiger Weise der Endoskopkamera 20 zugeordnet oder mit dieser verbunden sein. Alternativ kann die Mikrofoneinheit 23 auch als mobile Einheit ausgebildet sein, welche beispielsweise im Kopfbereich des Operateurs lösbar befestigen werden kann. Beispielsweise kann die Mikrofoneinheit 23 in Form eines drahtlosen "Headsets" oder auch in Form eines im Operationssaal angeordnetes Richtmikrofon realisiert sein. Die von der Mikrofoneinheit 23 erfassten Sprachbefehle SB und/oder Sprachbefehlskombinationen SBK werden vorzugsweise über eine drahtlose Datenverbindung an die Steuereinheit CU übertragen und in dieser über eine geeignete Schnittstelleneinheit an die Prozessoreinheit CPU zur weiteren Verarbeitung übergeben.

Die in der Steuereinheit CU implementierte "Sprachsteuerung" ermöglicht dem Operateur die Steuerung der Führung der Endoskopkamera 20 bzw. Nachführung der Endoskopkamera 20 nach dem Operationsinstrument 30, 30a, 30b mittels Eingabe von Sprachbefehlen SB und/oder Sprachbefehlskombinationen SBK. Der Einsatz einer "Sprachsteuerung" zur zumindest teilweisen Steuerung bzw. Nachführung einer Endoskopkamera 20 eines Operations-Assistenz-Systems 1 ist prinzipiell bekannt, wobei hierzu die Eingabe von vorgegeben Steuerbefehlen SB und/oder Sprachbefehlskombinationen SBK erforderlich ist, welche vorzugsweise eine Steuerung über ein Bedienelement mit vorgegebene Bewegungsrichtungen abbilden. Im Zuge dessen ist auch die Anpassung von Aufnahmeparametern der Endoskopkamera 20 an sich veränderte Operationsbedingungen durch die Eingabe vorgegebener Sprachbefehle SB möglich. Nachteilig ist bei bekannten Systemen jedoch keine intuitive, d.h. an die aktuelle Operationsszene angepasste Sprachsteuerung möglich. Vielmehr ist die Spracheeingabe für den Operateur nach wie vor komplex oder die Eingabe erfolgt über einen Kameraführungsassistenten, der vom Operateur entsprechend angewiesen wird. Hier setzt die Erfindung an und stellt eine intuitivere und damit für den Operateur benutzerfreundliche Sprachsteuerung bereit.

Neben der Sprachsteuerroutine SSR ist erfindungsgemäß zumindest eine Bilderfassungsroutine BER in der Steuereinheit CU vorgesehen, die zur kontinuierlichen Erfassung der von der Endoskopkamera 20 bereitgestellten Bilddaten BD betreffend den Operationsraum 12 und darin ggf. befindlicher medizinischer Operationsinstrumente 30, 30a, 30b ausgebildet ist. Die Bilderfassungsroutine BER wird ebenfalls in der Prozessoreinheit CPU der Steuereinheit CU ausgeführt und dieser werden über eine weitere Schnittstelleneinheit die von der Endoskopkamera 20 bereitgestellten Bilddaten BD vorzugsweise kontinuierlich zur Weiterverarbeitung zur Verfügung gestellt.

Der Bilderfassungsroutine BER ist in der Steuereinheit CU erfindungsgemäß eine Bildauswerteroutine BAR zugeordnet, die die von Endoskopkamera 20 erfassten Bilder B bzw. die zugehörigen Bilddaten BD basierend auf statistischen und/oder selbstlernenden Methoden der künstlichen Intelligenz, insbesondere unter Verwendung eines neuronalen Netzes kontinuierlich auswertet und klassifiziert. Vorzugsweise erfolgt die Auswertung und Klassifikation der Bilddaten BD mittels geeigneter Muster- und/oder Farberkennungsalgorithmen, die vorzugsweise Teil eines neuronalen Netzes sind. Durch die Klassifikation der Bilddaten BD ist eine automatisierte Detektion von vorgegebenen Objekten im von der Endoskopkamera 20 aufgenommen Bild B möglich, über welche zusätzliche objekt- und/oder szenebezogenen Informationen OI, SI über die im Bild B dargestellte Operationsszene bestimmbar sind. Über die ebenfalls in der Prozessoreinheit CPU ausgeführte Bildauswerteroutine BAR kann beispielsweise ein im aktuell erfassten Bild B der Endoskopkamera 20 sichtbares Operationsinstrument 30, 30a, 30b identifiziert und anschließend dessen Position und/oder Orientierung in einem dem Bild B zugeordneten zweidimensionalen kartesischen Bildschirmkoordinatensystem BKS ermittelt werden. Hierzu werden die Koordinaten X, Y zumindest eines Markers oder eines Markerpunktes des Objektes, beispielsweise des Operationsinstrument 30, 30a, 30b im Bildschirmkoordinatensystem BKS bestimmt. Zusätzlich zu den Koordinaten X, Y eines Markers oder eines Markerpunktes des Objektes kann die Lage eines Objektes im Bild B auch mittels einem oder mehrerer Vektoren V beschrieben werden.

Somit wird den von der Endoskopkamera 20 erfassten Bilddaten BD, welche in zweidimensionaler Form als Bilder B auf der Monitoreinheit darstellbar sind, mittels der Bildauswerteroutine BAR ein zweidimensionales, kartesisches Bildschirmkoordinatensystem BKS zugeordnet. Das zweidimensionalen kartesische Bildschirmkoordinatensystem BKS weist hierzu eine erste horizontal verlaufende Bildachse X und eine zweite vertikal verlaufende Bildachse Y auf, wobei der Ursprung des zweidimensionalen kartesischen Bildschirmkoordinatensystems BKS vorzugsweise dem Zentrum des auf der Monitoreinheit angezeigten Bildes zugeordnet ist, d.h. fällt mit der Bildmitte zusammen.

Damit sind den auf der Monitoreinheit dargestellten, das Bild B ergebenden Bildpunkten oder Bildpunktbereichen Koordinaten X, Y im zweidimensionalen kartesischen Bildschirmkoordinatensystem BKS zuordenbar. Dies ermöglicht die Einteilung des von der Endoskopkamera 20 aufgenommenen Bildern B bzw. der zugehörigen Bilddaten BD in ein vorgegebenes Koordinatensystem BKS, um damit die Position einzelner Objekte, insbesondere der Operationsinstrumente 30, 30a, 30b durch Ermittlung der zugehörigen Koordinaten X, Y im zweidimensionalen kartesischen Bildschirmkoordinatensystems BKS bestimmen zu können. Objekte im Sinne der Erfindung können jedoch auch im Bild B ersichtliche Organe oder Teile davon sowie weitere medizinische Hilfsmittel wie Klammern, Schrauben oder Teile davon sein.

In einer Ausführungsvariante der Erfindung kann die Endoskopkamera 20 auch zur Erfassung von dreidimensionalen Bildern B des Operationsraumes ausgebildet sein, über welche eine zusätzliche Tiefeninformation gewonnen wird. Die von einer derartigen dreidimensionalen Endoskopkamera 20 bereitgestellten Bilddaten BD dienen zur Erzeugung von dreidimensionalen Bildern B, die beispielsweise auf einer entsprechend eingerichteten Monitoreinheit angezeigt werden. Zur Betrachtung der auf der Monitoreinheit dargestellten dreidimensionalen Bilder B kann die Verwendung einer 3D-Brille erforderlich sein. Der Aufbau und die Funktionsweise von dreidimensionalen Endoskopkameras 20 ist an sich bekannt.

Bei Verwendung einer dreidimensionalen Endoskopkameras 20 wird den Bilddaten BD ein dreidimensionales, kartesisches Bildschirmkoordinatensystem zugeordnet, d.h. das zweidimensionale kartesisches Bildschirmkoordinatensystem BKS wird um eine weitere Koordinatenachse, und zwar die Z-Achse ergänzt. Vorzugsweise fällt die Z-Achse mit der Längsachse des Endoskops 21 zusammen. Die Position eines Bildpunktes im dreidimensionalen Bild B wird im dreidimensionalen Bildschirmkoordinatensystem durch die Angabe der zugehörigen X-, Y- und Z-Koordinaten erfasst.

Für die Erkennung und zuverlässige Unterscheidung verschiedener Objekte, beispielsweise unterschiedlicher Operationsinstrumente 30, 30a, 30b oder Organe oder sonstiger medizinischer Hilfsinstrumente wie Operationsklammern etc. in den erfassten Bilddaten BD ist vorab eine Auswertung einer Vielzahl von realen Operationsszenarien erforderlich. Hierzu werden in klinischen Studien im Rahmen von real durchgeführten medizinischen, vorzugsweise minimal-invasiven Operationen so genannte "Trainingsdatensätze" erzeugt. Diese Trainingsdatensätze umfassend dabei eine Vielzahl an Bilddaten BD von realen operativen Eingriffen, die jeweils vor deren weiteren Verwendung annotiert werden, d.h. die verschiedenen, darin dargestellten Objekte, beispielsweise die bereits genannten Operationsinstrumente 30, 30a, 30b werden nach Art bzw. Typ klassifiziert, pixelgenau segmentiert und mehrere Marker bzw. Markerpunkte gesetzt, die den Aufbau und Position der Operationsinstrumente 30, 30a, 30b beschreiben. Zur Unterscheidung der unterschiedlichen Operationsinstrumente 30, 30a, 30b können bei den klinischen Studien die Operationsinstrumente 30, 30a, 30b mit individuellen Markern, beispielsweise Farbmarkern oder Codes versehen werden, welche an vorgegebenen Instrumententeilen und/oder - abschnitten angebracht werden. Die jeweils angebrachten Marker können in den Trainingsdatensätzen durch Anwendung von speziellen Muster- und/oder Farberkennungsalgorithmen detektiert werden und abhängig davon Art, Anzahl und/oder Position der im Bild B vorhandenen Operationsinstrumente 30, 30a, 30b ermittelt und klassifiziert werden.

Aus der Vielzahl der in klinischen Studien ermittelten und annotierten Bilddaten BD unter Verwendung von mit vorgegebenen Markern versehenen Operationsinstrumenten 30, 30a, 30b können dann mittels statischen und/oder selbstlernenden Methoden der künstlichen Intelligenz ("Deep Learning Methods"), insbesondere mittels tiefer Neuronaler Netze auch weitere charakteristische Merkmale der beispielhaft genannten Operationsinstrumente 30, 30a, 30b oder sonstiger Objekte bestimmt werden, die dann die "Wissensbasis" einer "selbstlernenden" Bildauswerteroutine BAR bilden. Die "selbstlernende" Bildauswerteroutine BAR wird über die Auswertung der Vielzahl an Trainingsdatensätzen "trainiert" und die mittels dieser detektierbaren Objekte, beispielsweise Operationsinstrumente 30, 30a, 30b entsprechend klassifiziert. Dies ermöglicht dann eine automatisierte Erkennung von Objekten im Kamerabild B mittels der "trainierten" Bildauswerteroutine BAR ohne an den Objekten zusätzliche Marker anzubringen, und zwar durch Auswertung bzw. Analyse der Bilddaten BD mittels der dem Bild B bzw. den zugehörigen Bilddaten BD entnehmbaren Marker oder Markerpunkte der Objekte für sich genommen.

Auch kann darüber beispielsweise eine zumindest teilautomatisierte Verfolgung der Objekte während des operativen Eingriffs implementiert werden, in dem die Bewegungshistorie als auch die Bewegungsgeschwindigkeit eines im Bild B detektierten Objektes, beispielsweise eines Operationsinstrumentes 30, 30a, 30b ausgewertet und zur zumindest teilautomatisierten Steuerung der Endoskopkamera 20 verwendet wird. Die hierzu eingesetzten selbstlernenden Methoden bzw. Algorithmen ermöglichen dabei nicht nur die Verarbeitung von strukturierten, sondern auch unstrukturierten Daten wie beispielsweise den vorliegenden Bilddaten BD und insbesondere auch Sprachdaten SD.

Ein charakteristisches Merkmal eines Operationsinstrumentes 30, 30a, 30b kann beispielsweise ein eindeutiger Punkt eines Operationsinstrumentes 30, 30a, 30b wie die Instrumentenspitze S, Sa, Sb oder ein Gelenkpunkt zwischen dem Manipulator und dem Instrumentenschaft sein, welcher ohne zusätzliche angebrachte Marker als Markerpunkt des Operationsinstrumentes 30, 30a, 30b für sich genommen mittels der "trainierten" Bildauswerteroutine BAR detektierbar ist. Beispiele für unterschiedliche zu erkennende und entsprechend in der Bildauswerteroutine BAR klassifizierten Operationsinstrumente 30, 30a, 30b sind beispielsweise eine Zange, eine Schere, ein Skalpell, usw. Auch können charakteristische Merkmale von Organen oder weiteren medizinischen Hilfsmitteln als Marker bzw. Markerpunkte dienen.

Auch kann ein Marker oder Markerpunkte durch den Flächenschwerpunkt des im Bild dargestellten Flächenabschnitt eines Objektes, beispielsweise Organes oder Operationsinstruments 30, 30a, 30b gebildet sein. Insbesondere bei länglichen Objekten wie beispielsweise Operationsinstrumenten 30, 30a, 30b kann auch neben der Instrumentenspitze S, Sa, Sb als weiterer Marker oder Markerpunkte ein entlang der Längsachse des Operationsinstrumentes 30, 30a, 30b im Bild B verlaufender bzw. orientierter Vektor V erfasst werden, welcher die räumliche Orientierung des länglichen Objektes, insbesondere des Operationsinstrumentes 30, 30a, 30b im Bild B bzw. Bildkoordinatensystem BKS anzeigt.

Von besonderer Bedeutung ist u.a. auch eine zuverlässige Unterscheidung von mehreren im Bild B dargestellten Objekten, beispielsweise unterschiedlichen Operationsinstrumenten 30, 30a, 30b und/oder Organen und/oder weiteren medizinischen Hilfsmitteln, die sich im Bild B teilweise überlagern oder überlappen. Hier kann sich beispielsweise ein Instrument 30a im Erfassungsbereich der Endoskopkamera 20 oberhalb oder unterhalb eines anderen Instrumentes 30b befinden oder teilweise von einem Organ verdeckt sein. Insbesondere bei Verwendung von dreidimensionalen Endoskopkameras 20 ist aufgrund der zusätzlich in den Bilddaten BD enthaltenen Tiefeninformation eine Unterscheidung auch von überlappenden Objekten zuverlässig möglich.

Unter Berücksichtigung der genannten Rahmenbedingungen werden mittels der "trainierten" Bildauswerteroutine BAR die während eines operativen Eingriffs von der Endoskopkamera 20 in Form von Bilddaten BD erfassten Bilder B basierend auf statistischen und/oder selbstlernenden Methoden der künstlichen Intelligenz, insbesondere mittels eines neuronalen Netzes kontinuierlich ausgewertet und klassifiziert. Erfindungsgemäß ist die Erfassung sowohl zweidimensionaler als auch dreidimensionaler Bilder B anhand der Bilddaten BD möglich. Durch die kontinuierliche Auswertung und Klassifikation der Bilddaten BD werden objekt- und/oder szenebezogene Informationen OI, SI über die von der Endoskopkamera 20 aktuell im Bild B erfasste Operationsszene ermittelt und von der Sprachsteuerroutine SSR erfasste, d.h. aktuell eingegebene Sprachbefehle SB und/oder Sprachbefehlskombinationen SBL in Form von Sprachdaten SD abhängig von der erfassten Operationsszene, d.h. unter Berücksichtigung der ermittelten objekt- und/oder szenebezogenen Informationen OI, SI ausgewertet, d.h. eine kontextabhängige Sprachführung bzw. Sprachsteuerung der Führung der Endoskopkamera 20 über das erfindungsgemäße Operations-Assistenz-Systems 1 bereitgestellt.

Die erfindungsgemäße Sprachsteuerroutine SSR ist nicht nur zur Auswertung von vorgegebenen Sprachbefehlen SB und/oder Sprachbefehlskombinationen SBK mit vorgegebenen Inhalten wie beispielsweise Richtungsinformationen oder Betragsinformationen, sondern auch zur Auswertung von Sprachdaten SD mit objekt-und/oder szenenbezogenen Inhalten, nachfolgend als objekt- und/oder szenenbezogene Sprachbefehle OSB, SSB bezeichnet, eingerichtet. Hierzu ist die erfindungsgemäße Sprachsteuerroutine SSR zur kontinuierlichen Auswertung der erfassten Sprachdaten SD und ggf. auch Klassifizierung der darin enthaltenen objekt- und/oder szenenbezogenen Sprachbefehlen OSB, SSB basierend auf statistischen und/oder selbstlernenden Methoden der künstlichen Intelligenz, insbesondere unter Verwendung eines neuronalen Netzes eingerichtet.

Zusätzlich zur Erfassung der Bilddaten BD im Rahmen der durchgeführten klinischen Studien werden auch eine Vielzahl vom Operateur beispielsweise an den Kameraführungsassistenten gegebene Sprachbefehle SB erfasst und mittels statischen und/oder selbstlernenden Methoden der künstlichen Intelligenz ("Deep Learning Methods"), insbesondere mittels tiefer neuronaler Netze daraus charakteristische Sprachmerkmale bestimmt, die dann einen "Sprachwortschatz" einer "selbstlernenden" Sprachsteuerroutine SSR bilden. Die "selbstlernende" Sprachsteuerroutine SSR wird ebenfalls über die Auswertung der Vielzahl an durch die klinischen Studien erhaltenen Sprachbefehlsätze "trainiert" und daraus Sprachbefehlsklassen gebildet. Eine derartige "selbstlernende" Sprachsteuerroutine SSR ist somit in der Lage eine oder mehrere phonetische Lautfolge(n) nebst Sprechpausen zu erfassen, und zwar kann eine erfasste phonetische Lautfolge mittels des trainierten neuronalen Netzes dann durch Anwendung von Wort- und/oder Silbenerkennungsalgorithmen entsprechende im Sprachbefehl SB oder einer Sprachbefehlskombination SBK enthaltene Wörter oder Wortkombinationen ermitteln. Die Erkennung und der Vergleich mit bereits im "Sprachwortschatz" der "selbstlernenden" Sprachsteuerroutine SSR hinterlegten Wörter oder Wortkombinationen kann beispielsweise vektorbasiert erfolgen.

Die durch Analyse der Bilddaten BD gewonnen objekt- und/oder szenebezogenen Informationen OI, SI werden zur Auswertung Sprachdaten SD mit objekt- und/oder szenenbezogenen Inhalten verwendet und von der Sprachsteuerroutine SSR in entsprechende Steuersignale SS umgesetzt. Unter einem objektbezogene Sprachbefehl OSB wird ein Wort oder eine Wortkombination im Sprachbefehl SB bzw. einer Sprachbefehlskombination SBK verstanden, die auf im Bild B dargestellte Objekte Bezug nimmt bzw. nehmen. Analog dazu betreffen szenenbezogenen Sprachbefehle SSB ebenfalls Wörter oder Wortkombinationen in einem Sprachbefehle SB bzw. einer Sprachbefehlskombination SBK, die auf im Bild B dargestellte und ebenfalls von der Bildauswerteroutine BAR erkannte oder abgeleitete Operationsszenen, beispielsweise die aktuelle Lage bzw. Position eines oder mehrerer Objekte im Bild B Bezug nehmen.

Der Operateur kann somit mittels vom Kontext der aktuellen Operationsszene abgeleitete, objekt- und/oder szenenbezogene Sprachbefehle OSB, SSB wie beispielsweise *"Zeige das rechte Operationsinstrument"* oder *"Zeige das Skalpell"* oder *"Zeige die Gallenblase"* eine kontextabhängige bzw. szenenabhängige Sprachsteuerung durchführen.

Bei Eingabe dieser beispielhaft aufgeführten, mehrere Wörter umfassende objekt- und szenenbezogenen Sprachbefehlen OSB, SSB wird beispielsweise die Endoskopkamera 20 derart mittels des Operations-Assistenz-Systems 1 geführt oder nachgeführt, dass sich im Bild B das "Operationsinstrument" bzw. das "Skalpell" bzw. die "Gallenblase" von der rechten Bildhälfte in die Bildmitte verschiebt und ggf. auch vergrößert oder verkleinert dargestellt wird. Neben einer entsprechenden Führung der Endoskopkamera 20 ist somit auch eine Sprachsteuerung ausgewählter Kamerafunktionen wie beispielsweise "Zoomen" oder die Aktivierung von speziellen Bildfiltern möglich.

Voraussetzung hierfür ist jedoch eine zuverlässige Objekterkennung und eine entsprechende Zuordnung der erkannten bzw. detektierten Objekte im Bild B über die Ermittlung der zugehörigen Koordinaten X, Y von Markern oder Markerpunkten des jeweiligen Objektes im Bildschirmkoordinatensystem BKS. Das durch Anwendung von Methoden der künstlichen Intelligenz gewonnene Wissen über die im Bild B aktuell dargestellte Operationsszene wird erfindungsgemäß zur kontextabhängigen bzw. szeneabhängigen Sprachsteuerung des Operations-Assistenz-Systems 1 verwendet, um eine für den Operateur benutzerfreundliche Führung der Endoskopkamera 20 durch die Möglichkeit der Verwendung von objektbezogenen und/oder szenebezogenen Sprachbefehlen OSB, SSB während des operativen Eingriffs, d.h. in Echtzeit zu erreichen.

Hierzu ist die Sprachsteuerroutine SSR zur Erfassung und Auswertung von komplexen Sprachbefehlen SB bzw. Sprachbefehlskombinationen SBK eingerichtet, wobei eine Sprachbefehlskombination SBK mehrere Wörter oder mehrere Wortbestandteile aufweist, die durch einen oder mehrere Sprachbefehle bzw. Sprachkommandos SB, OSB, SSB gebildet sein können. Ein Wort oder mehrere Wörter können somit einen eigenen Sprachbefehl SB, OSB, SSB oder einen Teil eines Sprachbefehls SB, OSB, SSB bilden und eine Sprachbefehlskombination SBK kann auch mehrere derartiger Sprachbefehle SB, OSB, SSB umfassen.

Bei der Eingabe einer mehrere Wörter umfassenden Sprachbefehlskombination SBK durch den Operateur ist es erforderlich, dass die Eingabe der Wörter einer Sprachbefehlskombination SBK zwar mit Sprechpausen, aber zeitlich unmittelbar aufeinander folgend und innerhalb eines vorgegebenen Zeitintervalls durchgeführt wird. Die Sprachbefehlskombinationen SBK können dabei neben den erfindungsgemäßen objektbezogenen und/oder szenebezogenen Informationen OI, SI auch Steuerinformationen wie beispielsweise eine Richtungsinformation RI, eine Geschwindigkeitsinformation VI und/oder eine Betragsinformation BI umfassen. Letztgenannte Steuerinformationen betreffen an sich bekannte Steuerkommandos bezogen auf die Bewegung der Endoskopkamera 20 im Raumkoordinatensystems RKS. Die vom Operateur eingegebenen Sprachbefehle SB bzw. Sprachbefehlskombination SBK werden in Form von Sprachdaten SD weiterverarbeitet.

Die von Sprachsteuerroutine SSR erfassten Sprachdaten SD werden in Abhängigkeit der objekt- und/oder szenebezogenen Informationen OI, SI, welche von der Bildauswerteroutine BAR entweder aktuell zur Verfügung gestellt werden und/oder bereits in der Speichereinheit MU als "Wissensbasis" hinterlegt sind, ausgewertet. Ein in den Sprachdaten SD enthaltener objektbezogener Sprachbefehl OSB betrifft zumindest eine objektbezogene Information OI, wobei unter einer objektbezogenen Information OI im erfindungsgemäßen Sinne ein im Bild B dargestelltes Objekt oder eine auf dieses Objekt bezogene Information verstanden wird. Ein in den Sprachdaten SD enthaltener szenebezogener Sprachbefehl SSB ist auf eine szenebezogene Information SI gerichtet, wobei unter einer szenebezogenen Information beispielsweise die Positionierung eines oder mehrerer Objekte im Bild B bzw. im zugeordneten Bildkoordinatensystem BKS oder die Operationsszene als solche oder dieser zugeordneten Begriffe, insbesondere Fachbegriffe verstanden werden.

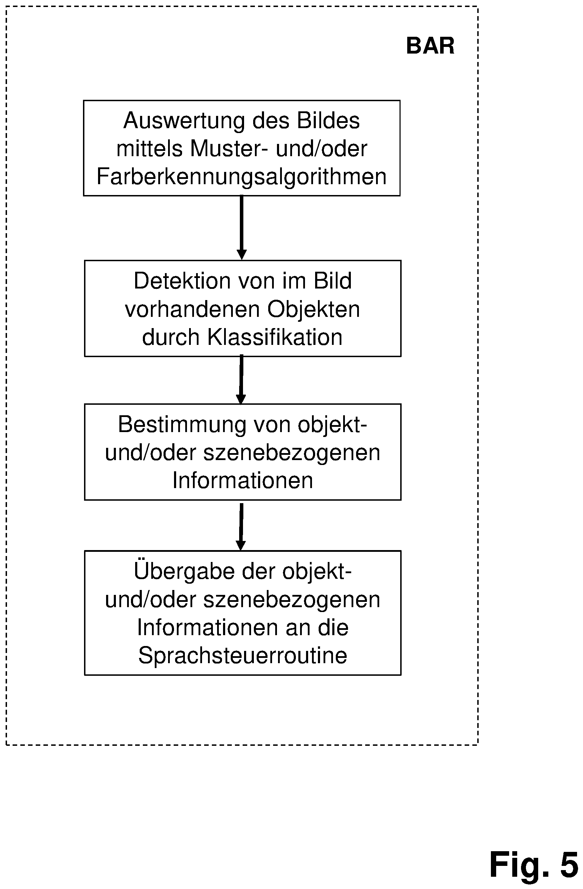

Figur 5 zeigt beispielhaft ein schematisches Ablaufdiagramm der in der Steuereinheit CU ausgeführten erfindungsgemäße Bildauswerteroutine BAR, der vorzugsweise ein trainiertes neuronales Netzwerk zur Auswertung der Bilddaten BD zugrunde liegt, über welche die zur kontextabhängigen Sprachsteuerung verwendeten objekt- und/oder szenebezogenen Informationen OI, SI gewonnen werden.

Nach erfolgter Bereitstellung des aktuell von der Endoskopkamera 20 erfassten Bildes B in Form von Bilddaten BD werden diese in der Steuereinheit CU von der Bildauswerteroutine BAR ausgewertet, und zwar werden die Bilddaten BD mit Hilfe der in der Bildauswerteroutine BAR implementierten Muster- und/oder Farberkennungsalgorithmen analysiert bzw. ausgewertet und im Bild B vorhandene Objekte oder Teile davon, insbesondere Operationsinstrumente 30, 30a, 30b, oder andere medizinische Hilfsmittel oder Organe detektiert. Die Detektion erfolgt dabei basierend auf einem der Bildauswerteroutine BAR zugrundliegenden, trainierten neuronalen Netzwerk, über das den einzelnen Objekten, insbesondere den Operationsinstrumenten 30, 30a, 30b Markern bzw. Markerpunkte wie beispielsweise eine Instrumentenspitze S, Sa, Sb, besondere Farb- oder Materialeigenschaften des Objektes oder das Gelenk zwischen Manipulator und Instrumentenschaft eines Operationsinstruments 30, 30a, 30b zugeordnet sind, und zwar durch entsprechende Analyse der Bilddaten BD.

Abhängig von den jeweils erkannten Markern oder Markerpunkten erfolgt dann eine Klassifizierung der Operationsszene und/oder der darin vorgefundenen Objekte, d.h. eine Bestimmung von objektbezogenen und/oder szenebezogenen Informationen OI, SI aus den analysierten bzw. ausgewerteten Bilddaten BD. Über das neuronale Netz werden hierbei vorzugsweise Folgen und/oder Kombinationen von Markern oder Markerpunkten geprüft.

Beispielsweise können im Zuge dessen die Art, der Typ, die Eigenschaften und/oder die Lage der im Bild B aufgefundene Objekte bestimmt werden. Eine entsprechend gewonnene Information betreffend ein klassifiziertes Objekt wird dann in Form einer objektbezogene Information OI entweder an die Sprachsteuerroutine SSR zur davon abhängigen Auswertung von eingehenden Sprachbefehlen SB übergeben. Zusätzlich kann die objektbezogene Information OI auch in der Speichereinheit MU gespeichert werden.

Eine objektbezogene Information OI ist beispielsweise die Art des dargestellten Operationsinstrumentes 30, 30a, 30b, d.h. welches konkrete Operationsinstrument 30, 30a, 30b im Bild B dargestellt ist, beispielsweise eine Schere oder eine Zange. Auch können weitere charakteristische Marker bzw. Markerpunkte für dieses konkrete Operationsinstrument 30, 30a, 30b bereits im trainierten neuronalen Netzwerk hinterlegt sein, über welche weitere objektbezogene Information OI und/oder szenebezogene Informationen SI abgeleitet werden können. Ist beispielsweise die Instrumentenspitze S, Sa, Sb einer als Schere klassifizierten Operationsinstrument 30, 30a, 30b als Marker oder Markerpunkt definiert, so kann daraus die Lage der Instrumentenspitze S, Sa, Sb und damit implizit auch die Lage des als Schere klassifizierten Operationsinstruments 30, 30a, 30b im Bild B als szenebezogene Information SI ermittelt werden.

Hierzu ist eine eindeutige Zuordnung der Position der Marker oder Markerpunkte des detektierten Operationsinstruments 30, 30a, 30b und damit der Position des Operationsinstrumentes im Bildschirmkoordinatensystem BKS erforderlich. Die Bestimmung der Position einzelner Marker bzw. Markerpunkte des detektierten Operationsinstruments 30, 30a, 30b erfolgt dabei durch die Ermittlung der entsprechenden Koordinaten Xa, Ya, Xb, Yb im Bildschirmkoordinatensystem BKS. Abhängig vom detektierten Objekttyp bzw. der zuordneten Marker oder Markerpunkte ist die Position des detektierten Objektes, im vorliegenden Fall des detektierten Operationsinstruments 30, 30a, 30b im Bild B über die zugehörigen Koordinaten Xa, Ya, Xb, Yb im Bildkoordinatensystem BKS eindeutig bestimmbar. In Figur 3 sind beispielhaft die Koordinaten Xa, Ya, Xb, Yb der jeweiligen Spitze Sa, Sb des ersten und zweiten Operationsinstruments 30a, 30b im Bildschirmkoordinatensystem BKS eingezeichnet.

Abhängig den Koordinaten Xa, Ya, Xb, Yb im Bildschirmkoordinatensystem BKS kann dann beispielsweise der Abstand Aa, Ab des Operationsinstruments 30, 30a, 30b zur Mitte des Bildes B bzw. zum Ursprung des Bildschirmkoordinatensystems BKS ermittelt bzw. berechnet werden. Vorzugsweise werden hierzu die Abstände Aa, Ab von vorgegebenen Markern bzw. Markerpunkten, wie beispielsweise der Instrumentenspitze S, Sa, Sb zur Bildmitte bzw. Ursprung des Bildschirmkoordinatensystems BKS bestimmt, um szenebezogenen Informationen SI über die Lage und/oder Position des Operationsinstruments 30, 30a, 30b im Bild B zu erhalten. Unter Verwendung einer dreidimensionalen Endoskopkamera 20 können zusätzlich auch noch weitere, nicht in den Figuren dargestellte Koordinaten betreffend eine Tiefeninformation in einem eine zusätzliche z-Achse umfassenden dreidimensionalen Bildschirmkoordinatensystem erfasst werden, über welche auch der Abstand zweier Objekte im Operationsraum 12 zueinander ermittelt und als weiteres Regel-und/oder Steuerkriterium verwendet werden kann.

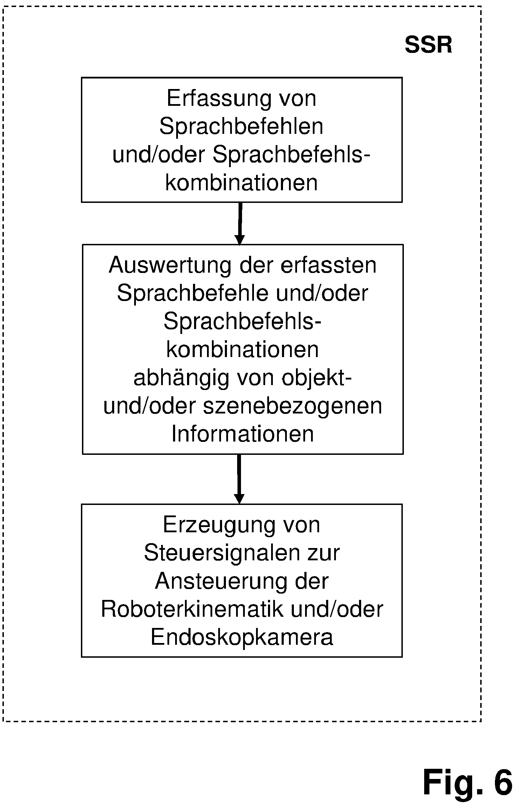

Die von den durch die Bildauswerteroutine BAR bereitgestellten objekt- und/oder szenebezogenen Informationen OI, SI werden in der Sprachsteuerroutine SSR zur Auswertung der erfassten Sprachbefehle oder Sprachkommandos SB, insbesondere von objekt- und/oder szenebezogenen Sprachbefehle OSB, SSB verwendet. Figur 6 zeigt beispielhaft ein schematisches Ablaufdiagramm einer erfindungsgemäßen Bildauswerteroutine BAR.

Nach der Erfassung der aktuell vom Benutzer bzw. Operateur eingegebenen Sprachbefehle SB und/oder Sprachbefehlskombinationen SBK in Form von Sprachdaten SD, welche insbesondere auch objekt- und/oder szenebezogenen Sprachbefehle OSB, SSB umfassen können, werden diese abhängig von den bereitgestellten objekt- und/oder szenebezogenen Informationen OI, SI ausgewertet. Damit besteht ein unmittelbarer zeitlicher Bezug zwischen den aktuell dem Operateur angezeigten Bild B, den davon abgeleiteten objekt-und/oder szenebezogenen Informationen OI, SI sowie den aktuell eingegebenen objekt-und/oder szenebezogenen Sprachbefehlen OSB, SSB. Ausgehend davon werden dann die Steuersignale SS zur entsprechenden Ansteuerung der Roboterkinematik des Operations-Assistenz-Systems 1 und/oder der Endoskopkamera 20 erzeugt.

Wird vom Operateur beispielsweise als Sprachbefehlskombination SBK die Wortkombination *"Zeige das rechte Operationsinstrument"* eingegeben, so werden die dadurch erhaltenen Sprachdaten SD dahingehend ausgewertet, ob zwischen den eingegebenen Wörtern *"Zeige", "das", "rechte", "Operationsinstrumente",* deren Reihenfolge und/oder zwischen diesen vorliegenden Sprachpausen und den ermittelten objekt- und/oder szenebezogenen Informationen OI, SI ein Zusammenhang besteht. Unter Verwendung eines entsprechenden trainierten neuronalen Netzes, welches Teil der Sprachsteuerroutine SSR ist, und der durch dies

Hierzu erfolgt ein Vergleich der in den Sprachdaten SD enthaltenen phonetischen Laut-und/oder Silbenfolgen mit dem in der Sprachsteuerroutine SSR und/oder in der Speichereinheit MU hinterlegten Merkmalsfolgen oder Merkmalskombinationen des Sprachwortschatzes und bei Übereinstimmung einzelner Merkmalsfolgen oder Merkmalskombinationen wird ein zugeordneter objekt- und/oder szenebezogenen Sprachbefehlen OSB, SSB erkannt und ein vorgegebenes oder davon abgeleitetes Steuersignal SS erzeugt. Beispielsweise können zusätzlichen zu den objektbezogenen Information OI betreffend das klassifizierte Objekt "Operationsinstrument" szenebezogene Informationen SI in Form der Lage des klassifizierten Objektes "Operationsinstrument" in Bezug auf die Mitte des Bildes B hinterlegt sein, die als Referenzwörter Positionsangaben wie beispielsweise "oben", "unten", "links", "rechts", jeweils bezogen auf die Bildmitte umfassen.

Bei einem im Bild B rechts von der Bildmitte befindlichen Operationsinstrument werden daher in der Sprachsteuerroutine SSR aufgrund der bereitgestellten objekt- und/oder szenebezogenen Informationen OI, SI die Wörter "rechts" und "Operationsinstrument" als objekt- und/oder szenebezogenen Sprachbefehlen OSB, SSB für die Auswertung der erfassten Sprachdaten SD bereitgestellt. Dem weitere Sprachbefehl "Zeige" ist ebenfalls über das neuronale Netz der Sprachsteuerroutine SSR ein Sprachbefehl SB zugeordnet, wobei das davon abgeleitete Steuerkommando SS abhängig von den weiteren objekt-und/oder szenebezogenen Sprachbefehlen OSB, SSB "rechts" und "Operationsinstrument" ist. Die Sprachsteuerroutine SSR wertet noch weitere, von der Bildauswerteeinheit BAR bereitgestellte szenebezogene Informationen SI aus, und zwar den im Bildkoordinatensystem angegebenen Koordinaten Xb, Yb der Spitze Sb des "rechts" von der Bildmitte dargestellten zweiten Operationsinstrumentes 30b sowie den errechneten Abstand Ab der Spitze Sb zur Bildmitte bzw. zum Ursprung des Bildkoordinatensystems BKS. Alternativ kann auch ein dem zweiten Operationsinstrument 30b zugeordneter Vektor V ausgewertet werden.

Abhängig von den genannten objekt- und/oder szenebezogene Informationen OI, SI und der durch Eingabe des Sprachbefehle *"Zeige das rechte Operationsinstrument"* erfassten Sprachdaten SD werden von der Sprachsteuerroutine SSR die zugeordneten Steuersignale SS erzeugt, über welche die Endoskopkamera 20 mittels des Operations-Assistenz-Systems 1 derart verfahren wird, dass die Spitze Sb des zweiten Operationsinstrumentes 30b im Bild B der Endoskopkamera 20 in der Bildmitte bzw. im Ursprung des Bildkoordinatensystems BKS zu liegen kommt.

Die von der Bildauswerteroutine BAR kontinuierlich bereitgestellten objekt- und/oder szenebezogene Informationen OI, SI werden entsprechend der Sprachsteuerroutine SSR zur Verarbeitung der erfassten Sprachbefehle SB ebenfalls kontinuierlich zur Verfügung gestellt.

Auch kann die Bildauswerteroutine BAR und/oder die Sprachsteuerroutine SSR derart eingerichtet sein, dass durch das jeweilige neuronale Netz eine Berücksichtigung der Operationshistorie bei der Auswertung der Bild- und/oder Sprachdaten BD, SD berücksichtigt wird. Beispielsweise kann durch die Bildauswerteroutine BAR und/oder die Sprachsteuerroutine SSR der aktuelle Status eines minimal-invasiven operativen Eingriffs erfasst werden und die Endoskopkamera 20 derart ansteuern, dass das im nächsten Operationsschritt relevante "Field of Interest" automatisch, d.h. ohne Eingabe konkreter Sprachbefehle SB. Beispielsweise kann im Bild B bereits ein im Rahmen einer Gallenblasen-Operation ein Organ, beispielsweise eine offene Arterie ggf. vergrößert gezeigt werden, welche im nächsten Schritt beispielsweise mittels einer Operationsklammer zu verschließen ist.

In einer Ausführungsvariante wird die Sprachsteuerung über die Sprachsteuerroutine SSR ausschließlich bei Betätigung eines Aktivierungselementes, vorzugsweise eines Aktivierungsschalters oder -tasters durch den Operateur oder durch einen vorgegebenen Aktivierungssprachbefehl vom Operateur aktiviert. Dies dient insbesondere dazu, um eine ungewollte Eingabe von Sprachbefehlen SB durch den Operateur zu verhindern.

Auch können beispielsweise die aktuellen Koordinaten X, Y eines Referenzpunktes im Bild B bzw. Bildkoordinatensystem BKS durch entsprechende Spracheingabe des Operateurs in er Speichereinheit MU gespeichert werden, um das erneute Anfahren dieses Referenzpunktes im weiteren Verlauf der Operation zu vereinfachen.

Auch kann zusätzlich zur Bilderfassung über die Endoskopkamera 20 auch der Durchblutungszustand einzelner Organe, vorzugsweise unter Verwendung von Nahinfrarottechniken, erfasst und im Bild B angezeigt bzw. als erweitere Bilddaten mittels der Bilderfassungsroutine BER erfasst werden.

Die Erfindung wurde voranstehend an Ausführungsbeispielen beschrieben. Es versteht sich, dass zahlreiche Änderungen sowie Abwandlungen möglich sind, ohne dass dadurch der der Erfindung zugrundeliegende Erfindungsgedanke verlassen wird.

### Bezugszeichenliste

- 1: Operations-Assistenz-System
- 2: Basiseinheit
- 2.1: Trägerplatte
- 2.2: Basisgehäuse
- 2.3: Befestigungselement
- 3: Tragsäule
- 3': unteren Endabschnitt
- 3": oberer Endabschnitt
- 4: erster Roboterarm
- 4': erster Endabschnitt
- 4": zweiter Endabschnitt
- 5: zweiter Roboterarm
- 5': erster Endabschnitt
- 5": zweiter Endabschnitt
- 6: Hilfsinstrumententräger
- 7: abgewinkeltes Gelenkstück
- 8: Steuereinrichtung
- 10: Patientenkörper
- 11: erste Operationsöffnung ("Trokar")
- 12: Operationsraum
- 13: zweite Operationsöffnung ("Trokar")
- 20: Endoskopkamera
- 21: Endoskop
- 22: Kameraeinheit
- 23: Mikrofoneinheit
- 30: Operationsinstrument
- 30a: erstes Operationsinstrument
- 30b: zweites Operationsinstrument
- 30': freies Ende bzw. Griffbereich
- 30": freies Ende
- 30a": freies Ende des ersten Operationsinstruments
- 30b": freies Ende des zweiten Operationsinstruments
- 31: Griffelemente
- 32: Funktionselement(e)

- Aa, Ab: Abstand zum Ursprung
- B: Bild
- BAR: Bildauswerteroutine
- BER: Bilderfassungsroutine
- BD: Bilddaten
- BI: Betragsinformation
- BKS: Bildkoordinatensystem
- CU: Steuereinheit
- LI: Längsachse
- LK: Längsachse
- OI: objektbezogene Information
- OSB: objektbezogener Sprachbefehl
- RI: Richtungsinformation
- RKS: Raumkoordinatensystem
- S: Spitze des medizinischen Instrumentes
- Sa: Spitze des ersten Operationsinstruments
- Sb: Spitze des zweiten Operationsinstruments
- SA1: erste Schwenkachse
- SA2: zweite Schwenkachse
- SA3: dritte Schwenkachse
- SA4: vierte Schwenkachse
- SA5: fünfte Schwenkachse
- SB: Steuerbefehl
- SBK: Steuerbefehlskombination
- SD: Sprachdaten
- SI: szenebezogene Information
- SS: Steuersignale
- SSB: szenebezogener Sprachbefehl
- SSR: Sprachsteuerroutine
- VI: Geschwindigkeitsinformation
- x, y, z: Raumachsen des Raumkoordinatensystems
- X, Y: Raumachsen des Bildkoordinatensystems
- Xa, Ya: Koordinaten einer Instrumentenspitze
- Xb, Yb: Koordinaten einer Instrumentenspitze
- V: Vektor(en)
- Z: weitere Raumachse eines dreidimensionalen Bildkoordinatensystems

## Patentansprüche

1. Operations-Assistenz-System (1) zur Führung einer Endoskopkamera (20), welche über eine erste Operationsöffnung (11) in einem Operationsraum (12) eines Patientenkörpers (10) zumindest abschnittsweise einführbar und gesteuert bewegbar ist, umfassend eine Endoskopkamera (20) zur Erfassung von Bildern (B) des Operationsraumes (12) in Form von Bilddaten (BD) und eine die Endoskopkamera (20) mittels einer Hilfsinstrumentenhalterung (6) freiendseitig aufnehmende Roboterkinematik (3, 4, 5), die zur Führung der Endoskopkamera (20) im Operationsraum (12) motorisch gesteuert bewegbar ist, und zwar über von einer Steuereinheit (CU) erzeugte Steuersignale (SS), dass in der Steuereinheit (CU) zumindest eine Sprachsteuerroutine (SSR) ausgeführt wird, über die Sprachbefehle und/oder Sprachbefehlskombinationen (SB, SBK, OSB, SSB) in Form von Sprachdaten (SD) erfasst, ausgewertet und abhängig davon die Steuersignale (SS) erzeugt werden, und dass in der Steuereinheit (CU) zumindest eine Bilderfassungsroutine (BER) zur kontinuierlichen Erfassung der von der Endoskopkamera (20) bereitgestellten Bilddaten (BD) betreffend den Operationsraum (12) ausgeführt wird, **dadurch gekennzeichnet, dass** in der Steuereinheit (CU) zumindest eine Bildauswerteroutine (BAR) vorgesehen ist, mittels der die erfassten Bilddaten (BD) basierend auf statistischen und/oder selbstlernenden Methoden der künstlichen Intelligenz kontinuierlich ausgewertet und klassifiziert werden, dass mittels der kontinuierlichen Auswertung und Klassifikation der Bilddaten (BD) objekt-und/oder szenebezogene Informationen (OI, SI) betreffend die von der Endoskopkamera (20) aktuell im Bild (B) erfasste Operationsszene ermittelt werden, und dass die erfassten Sprachdaten (SD) abhängig von den erfassten objekt-und/oder szenebezogene Informationen (OI, SI) ausgewertet werden.

2. Operations-Assistenz-System nach Anspruch 1, **dadurch gekennzeichnet, dass** die Bildauswerteroutine (BAR) zur Auswertung der erfassten Bilddaten (BD) ein neuronales Netz mit Muster- und/oder Farberkennungsalgorithmen umfasst.

3. Operations-Assistenz-System nach Anspruch 2, **dadurch gekennzeichnet, dass** die Muster- und/oder Farberkennungsalgorithmen zur Erfassung oder Detektion von im Bild (B) vorhandener Objekte oder Teile davon, insbesondere von Operationsinstrumenten (30, 30a, 30b) oder anderen medizinischen Hilfsmitteln oder Organen eingerichtet und trainiert sind.

4. Operations-Assistenz-System nach einem der vorhergehenden Ansprüche, **dadurch gekennzeichnet, dass** die Sprachsteuerroutine (SSR) zur Auswertung der Sprachdaten (SD) basierend auf statistischen und/oder selbstlernenden Methoden der künstlichen Intelligenz eingerichtet ist.

5. Operations-Assistenz-System nach Anspruch 4, **dadurch gekennzeichnet, dass** die Sprachsteuerroutine (SSR) zur Auswertung der Sprachdaten (SD) ein neuronales Netz mit Laut- und/oder Silbenerkennungsalgorithmen umfasst.

6. Operations-Assistenz-System nach Anspruch 5, **dadurch gekennzeichnet, dass** die Laut- und/oder Silbenerkennungsalgorithmen zur Erfassung von in den Sprachdaten (SD) enthaltenen Laute, Silben, Wörtern, Wortpausen und/oder Kombinationen davon eingerichtet ist und/oder dass die Sprachsteuerroutine (SSR) zur einer von den objekt- und/oder szenebezogenen Informationen (OI, SI) abhängigen Auswertung der Sprachdaten (SD) eingerichtet ist.

7. Operations-Assistenz-System nach Anspruch 6, **dadurch gekennzeichnet, dass** die Sprachsteuerroutine (SSR) zur Erfassung von in den Sprachdaten (SD) enthaltenen objekt- und/oder szenebezogenen Sprachbefehlen (OSB, SSB) ausgebildet ist, wobei von der Sprachsteuerroutine (SSR) abhängig von den erfassten objekt- und/oder szenebezogenen Sprachbefehlen (OSB, SSB) zumindest ein Steuersignal (SS) erzeugt wird, über welches zumindest die Bewegung der Endoskopkamera (20) richtungs-, geschwindigkeits- und/oder betragsmäßig gesteuert wird.

8. Operations-Assistenz-System nach einem der Ansprüche 4 bis 7, **dadurch gekennzeichnet, dass** die Sprachsteuerroutine (SSR) auch zur Erfassung und Auswertung von Richtungs- und/oder Geschwindigkeitsinformationen (RI, VI) und/oder zugehöriger Betragsinformation (BI) in den Sprachdaten (SD) ausgebildet ist.

9. Operations-Assistenz-System nach einem der vorhergehende Ansprüche, **dadurch gekennzeichnet, dass** die Endoskopkamera (20) zur Erfassung eines zweidimensionalen oder eines dreidimensionalen Bildes (B) ausgebildet ist und/oder dass dem Bild (B) mittels der Bildauswerteroutine (BAR) ein zweidimensionales Bildkoordinatensystem (BKS) oder dreidimensionales Bildkoordinatensystem zugeordnet wird.

10. Operations-Assistenz-System nach Anspruch 10, **dadurch gekennzeichnet, dass** zur Ermittlung der Lage und/oder Position eines Objektes (B) im Bild (B) die Koordinaten (X, Y) des Objektes oder zumindest eines Markers oder eines Markerpunktes des Objektes im Bildschirmkoordinatensystem (BKS) bestimmt werden.

11. Operations-Assistenz-System nach einem der vorhergehenden Ansprüche, **dadurch gekennzeichnet, dass** mittels der Bildauswerteroutine (BAR) als Objekte oder Teile von Objekten im Bild (B) dargestellte Operationsinstrumente (30, 30a, 30b) und/oder Organe und/oder andere medizinische Hilfsmittel detektiert werden.

12. Operations-Assistenz-System nach Anspruch 11, **dadurch gekennzeichnet, dass** zur Detektion von Objekten oder Teilen von Objekten ein oder mehrere Marker oder Markerpunkte eines Objektes mittels der Bildauswerteroutine (BAR) detektiert werden, wobei als Marker oder Markerpunkte beispielsweise eine Instrumentenspitze (S, Sa, Sb), besondere Farb- oder Materialeigenschaften des Objektes und/oder ein Gelenk zwischen einem Manipulator und dem Instrumentenschaft eines Operationsinstruments (30, 30a, 30b) verwendet werden.

13. Operations-Assistenz-System nach Anspruch 12, **dadurch gekennzeichnet, dass** mittels der Bildauswerteroutine (BAR) die detektierten Marker oder Markerpunkte zur Klassifizierung der Operationsszene und/oder der darin aufgefundenen Objekte(n) ausgewertet werden und abhängig davon die objektbezogenen und/oder szenebezogenen Informationen (OI, SI) bestimmt werden und dass die von der Bildauswerteroutine (BAR) bestimmten objektbezogenen und/oder szenebezogenen Informationen (OI, SI) an die Sprachsteuerroutine (SSR) übergeben werden.

14. Verfahren zur Erzeugung von Steuersignalen (SS) zur Ansteuerung einer motorisch gesteuert bewegbaren Roboterkinematik (3, 4, 5) eines Operations-Assistenz-Systems (1) zur Führung einer Endoskopkamera (20), bei dem die Endoskopkamera (20) mittels einer Hilfsinstrumentenhalterung (6) freiendseitig an der Roboterkinematik (3, 4, 5) angeordnet ist, bei dem die Endoskopkamera (20) über eine erste Operationsöffnung (11) zumindest abschnittsweise in den Operationsraum (12) eines Patientenkörpers (10) einführbar ist und bei dem in einer Steuereinheit (CU) zumindest eine Sprachsteuerroutine (SSR) zur Erzeugung der Steuersignale (SS) ausgeführt wird, wobei mittels der Sprachsteuerroutine (SSR) Sprachbefehle und/oder Sprachbefehlskombinationen (SB, SBK, OSB, SSB) in Form von Sprachdaten (SD) erfasst, ausgewertet und abhängig davon die Steuersignale (SS) erzeugt werden, und bei dem in der Steuereinheit (CU) zumindest eine Bilderfassungsroutine (BER) zur kontinuierlichen Erfassung der von der Endoskopkamera (20) bereitgestellten Bilddaten (BD) betreffend den Operationsraum (12) ausgeführt wird, **dadurch gekennzeichnet, dass** mittels einer in der Steuereinheit (CU) ausgeführten Bildauswerteroutine (BAR) die erfassten Bilddaten (BD) basierend auf statistischen und/oder selbstlernenden Methoden der künstlichen Intelligenz kontinuierlich ausgewertet und klassifiziert werden, dass mittels der kontinuierlichen Auswertung und Klassifikation der Bilddaten (BD) objekt- und/oder szenebezogene Informationen (OI, SI) betreffend die von der Endoskopkamera (20) aktuell im Bild (B) erfasste Operationsszene ermittelt werden, und dass die erfassten Sprachdaten (SD) abhängig von den erfassten objekt- und/oder szenebezogene Informationen (OI, SI) ausgewertet werden.

15. Verfahren nach Anspruch 14, **dadurch gekennzeichnet, dass** in der Bildauswerteroutine (BAR) die erfassten Bilddaten (BD) mittels Muster- und/oder Farberkennungsalgorithmen eines neuronalen Netzes ausgewertet werden.

16. Verfahren nach Anspruch 15, **dadurch gekennzeichnet, dass** mittels der Muster-und/oder Farberkennungsalgorithmen die im Bild (B) dargestellten Objekte oder Teile von Objekten, insbesondere Operationsinstrumenten (30, 30a, 30b) oder anderen medizinischen Hilfsmitteln oder Organen detektiert werden und/oder dass zur Detektion der Objekte oder Teile von Objekten ein oder mehrere Marker oder Markerpunkte eines Objektes mittels der Bildauswerteroutine (BAR) detektiert werden, wobei als Marker oder Markerpunkte beispielsweise eine Instrumentenspitze (S, Sa, Sb), besondere Farb- oder Materialeigenschaften des Objektes und/oder ein Gelenk zwischen einem Manipulator und dem Instrumentenschaft eines Operationsinstruments (30, 30a, 30b) verwendet werden.

17. Verfahren nach Anspruch 16, **dadurch gekennzeichnet, dass** mittels der Bildauswerteroutine (BAR) die detektierten Marker oder Markerpunkte zur Klassifizierung der Operationsszene und/oder der darin aufgefundenen Objekte ausgewertet werden und abhängig davon die objektbezogenen und/oder szenebezogenen Informationen (OI, SI) bestimmt werden.

18. Verfahren nach Anspruch 17, **dadurch gekennzeichnet, dass** die von der Bildauswerteroutine (BAR) bestimmten objektbezogenen und/oder szenebezogenen Informationen (OI, SI) an die Sprachsteuerroutine (SSR) übergeben werden.

19. Verfahren nach einem der Ansprüche 14 bis 18, **dadurch gekennzeichnet, dass** in der Sprachsteuerroutine (SSR) die erfassten Sprachdaten (SD) mittels Laut- und/oder Silbenerkennungsalgorithmen eines neuronalen Netzes ausgewertet werden.

20. Verfahren nach Anspruch 19, **dadurch gekennzeichnet, dass** mittels der Laut-und/oder Silbenerkennungsalgorithmen in den Sprachdaten (SD) enthaltenen Laute, Silben, Wörtern, Wortpausen und/oder Kombinationen davon erfasst werden und/oder dass mittels der Sprachsteuerroutine (SSR) objekt- und/oder szenebezogene Sprachbefehle (OSB, SSB) erfasst und abhängig von den übergebenen objekt- und/oder szenebezogene Informationen (OI, SI) ausgewertet werden.

21. Verfahren nach einem der Ansprüche 14 bis 20, **dadurch gekennzeichnet, dass** dem Bild (B) mittels der Bildauswerteroutine (BAR) ein zweidimensionales Bildkoordinatensystem (BKS) zugeordnet wird und zur Lage- oder Positionsbestimmung eines Objektes (B) im Bild (B) die Koordinaten (X, Y) des Objektes oder zumindest eines Markers oder eines Markerpunktes des Objektes im Bildschirmkoordinatensystem (BKS) bestimmt werden.
